# EUROPEAN PATENT APPLICATION

(11) **EP 4 803 501 A1**
(43) Date of publication of application: **09.09.2026**
(21) Application number: 24884479.7
(22) Date of filing: 21.10.2024
(51) Int. Cl.: C07C 51/09, C07C 59/245

(54) **SYNTHESIS METHOD FOR BEMPEDOIC ACID AND BEMPEDOIC ACID INTERMEDIATE, AND BEMPEDOIC ACID INTERMEDIATE**

(30) Priority: 02.11.2023 CN 202311447890
(71) Applicant: Yangzhou Aurisco Pharmaceutical Co., Ltd, Yangzhou, Jiangsu 225100 (CN)
(72) Inventor: ZHAI, Ning, Yangzhou, Jiangsu 225100 (CN); WANG, Guoping, Yangzhou, Jiangsu 225100 (CN); ZHAO, Min, Yangzhou, Jiangsu 225100 (CN); YU, Zhenpeng, Yangzhou, Jiangsu 225100 (CN); WANG, Shikang, Yangzhou, Jiangsu 225100 (CN)
(74) Representative: Mewburn Ellis LLP
(86) International application number: PCT/CN2024/126031
(87) International publication number: WO 2025/092475

(57) **Abstract**

The present invention provides methods for preparing bempedoic acid and bempedoic acid intermediates, and bempedoic acid intermediates. The preparation method for bempedoic acid includes: subjecting a novel intermediate Compound 6 as a raw material to hydrolysis, removal of the hydroxyl protecting group, and reduction to obtain bempedoic acid. The preparation method for the intermediate Compound 6 includes: subjecting caprolactone as a starting material to self-condensation, ring opening, bromination, carbonyl protection, and α alkylation, to obtain Compound 6. The preparation method for bempedoic acid according to the present invention has simple and safe operation, high yield, and low production cost, thus being applicable to industrial production.

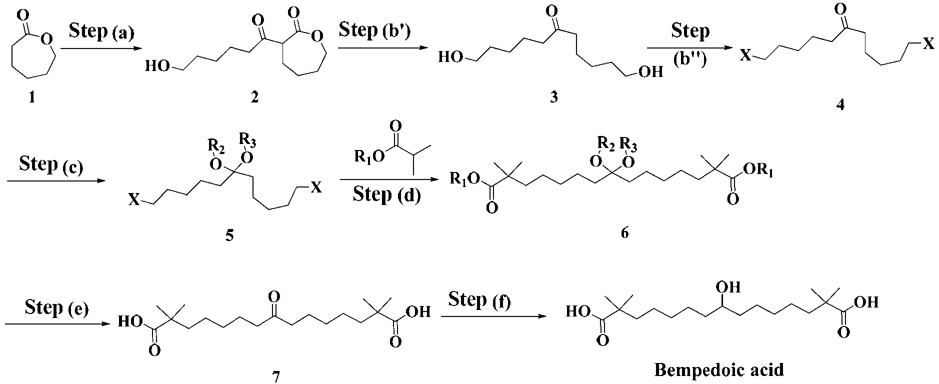

## Description

### TECHNICAL FIELD

The present invention relates to the field of preparation of organic compounds, and more specifically, to methods for preparing bempedoic acid and bempedoic acid intermediates, and bempedoic acid intermediates.

### BACKGROUND

Bempedoic acid is an adenosine triphosphate citrate lyase (ACL) inhibitor, which can reduce low-density lipoprotein cholesterol (LDL-C) by inhibiting cholesterol synthesis in the liver. The agent was approved by the US Food and Drug Administration (FDA) for marketing in the United States in February 2020. In recent 20 years, it is the first non-statin oral cholesterol-lowering drug approved by FDA for treating adult patients with heterozygous familial hypercholesterolemia or adult patients with atherosclerotic cardiovascular disease in need of further reduction of LDL-C. Bempedoic acid has a molecular structural formula shown below:

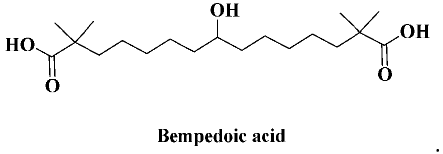

Currently, there are many reported synthetic routes for the synthesis of bempedoic acid in China and other countries. However, they have problems such as complicated process routes and high production costs. The synthetic route of bempedoic acid reported in the prior art WO2004067489 is Route 1 shown below:

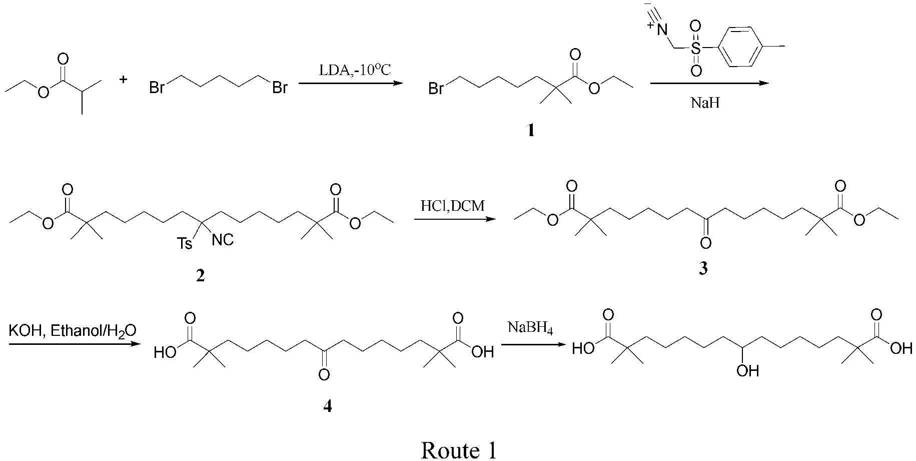

In this route, ethyl isobutyrate and 1, 5-dibromopentane are used as starting materials, which are condensed in the presence of lithium diisopropylamide (LDA) at a temperature to obtain ethyl 7-bromo-2,2-dimethylheptanoate (Compound 1). Compound 1 is used as an alkylating agent and reacted with tosylmethyl isocyanide (TosMIC) under a strong alkaline condition in the presence of tetrabutylammonium iodide (TBAI) as a catalyst to obtain Compound 2. Then, Compound 2 is hydrolyzed under an acidic condition to obtain Compound 3, and Compound 3 is hydrolyzed in an ethanol system to obtain Compound 4. Next, Compound 4 is reduced with NaBH₄, to finally obtain the target product bempedoic acid. α-alkylation in the first step of this process has poor selectivity, and disubstituted impurities cannot be avoided. Tosylmethyl isocyanide used in the second step is toxic and difficult to obtain, and the atomic economy is poor. Moreover, sodium hydride, a hazard material, is used, which is not conducive to the industrial production operation. Furthermore, excessive 1,5- dibromopentane is used to improve the selectivity, resulting in large residues of related impurities such as 1,5-dibromopentane, so rectification and purification are further needed. Potential genotoxic impurities (tosyl derivatives) is produced after hydrolysis in the third step, which is not conducive to the quality control of active pharmaceutical ingredients. In summary, this route has high loss and high potential risk, and is not suitable for use in industrial production.

The synthetic route of bempedoic acid reported in the prior art CN116396158 is Route 2 shown below:

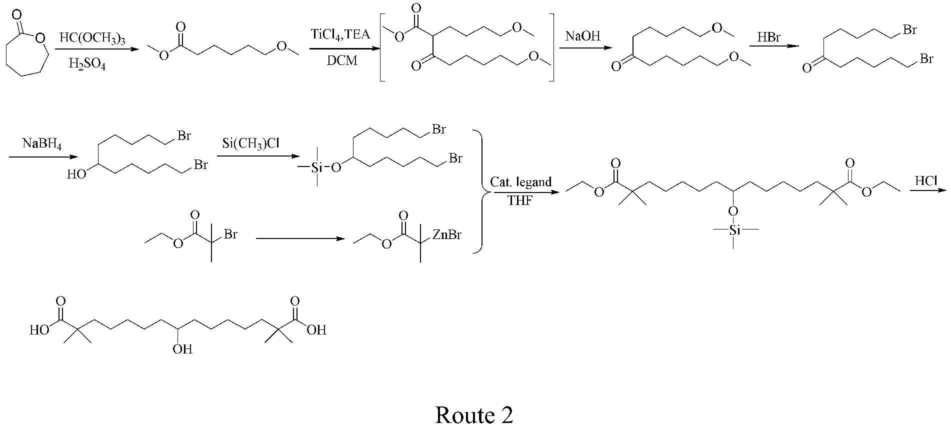

Caprolactone is used as a starting material in the route, which is subjected to ring opening, methylation, Dieckmann condensation in the presence of titanium tetrachloride as a catalyst, alkaline hydrolysis, decarboxylation, bromination, reduction with sodium borohydride, and protection with trimethylsilane (6 steps), to obtain a key intermediate 1,11-dibromoundecan-6-oxytrimethylsilyl ether. Then, the intermediate is coupled with (1-ethoxy-2-methyl-1-oxopropan-2-yl)zinc bromide to obtain diethyl 2,2,14,14-tetramethyl-8-(trimethylsilyloxy)pentadecandicarboxylate, which is finally hydrolyzed under an acidic condition and deprotected to obtain bempedoic acid. Although cheap caprolactone is used as the starting material in the route, the route is longer, and 2-bromoethyl isobutyrate used is expensive, thus greatly increasing the production cost.

The synthetic route of bempedoic acid reported in the prior art CN114907204 is Route 3 shown below:

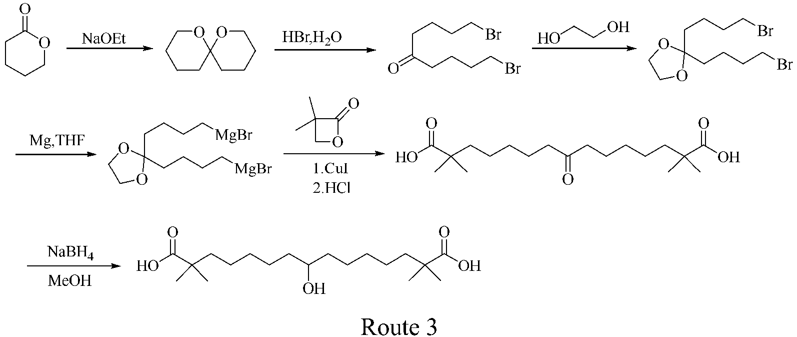

Valerolactone is used as a starting material in the route, which is subjected to Dieckmann condensation, bromination, protection with ethylene glycol, copper-catalyzed Grignard coupling, and reduction with sodium borohydride, to obtain the target compound bempedoic acid. The route is creative in design and simple in route, but 3,3-dimethyloxetan-2-one used is expensive, and difficult to obtain, the Grignard coupling reaction is difficult to control, and the yield is low, which limit the industrial application of this route.

In summary, it is of great significance to develop a synthetic route with simple and safe operation, low production cost, high yield and quality and practical industrial application value.

### SUMMARY

In an aspect, an object of the present invention is to provide a method for preparing bempedoic acid.

In a preferred embodiment, the method for preparing bempedoic acid includes the following steps:
(e) hydrolyzing Compound 6, and removing the carbonyl protecting group to obtain Compound 7; and
(f) reducing Compound 7 to obtain bempedoic acid. The reaction scheme is as follows: 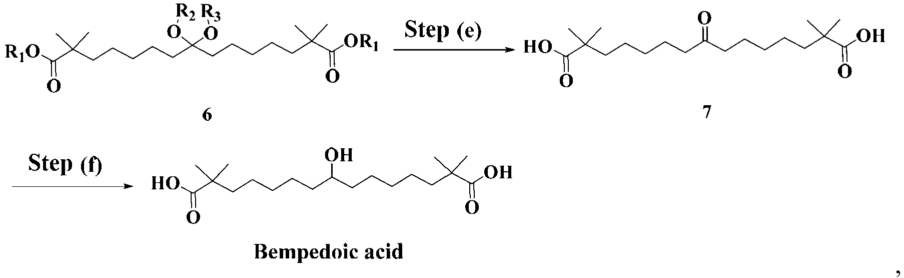
in which R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 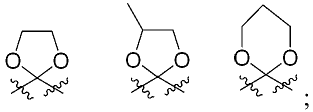 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, R₁ is selected from methyl or ethyl, and R₂ and R₃, together with the oxygen and carbon to which they are attached, form

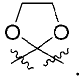

In another preferred embodiment, in Step (e), the hydrolysis is carried out under a basic condition, and the base is selected from NaOH, KOH, LiOH, Ba(OH)₂, Me₃SnOH, or a combination thereof, and more preferably, NaOH. In another preferred embodiment, in Step (e), the solvent used is selected from water, water/methanol or water/ethanol. In another preferred embodiment, in Step (e), the hydrolysis temperature is 25-120°C.

In another preferred embodiment, in Step (e), the removal of the carbonyl protecting group is carried out under an acidic condition, and the acid is selected from hydrochloric acid, sulfuric acid, sulfuric acid, hydrobromic acid, or a combination thereof, and more preferably, hydrochloric acid. In another preferred embodiment, in Step (e), the removal of the carbonyl protecting group is carried out at room temperature.

In another preferred embodiment, in Step (e), Compound 6 is removed of the carbonyl protecting group, and then hydrolyzed to obtain Compound 7.

In another preferred embodiment, in Step (f), the reducing agent used for the reduction is selected from sodium borohydride, potassium borohydride, lithium borohydride, sodium cyanoborohydride, or sodium triacetoxyborohydride, and more preferably, sodium borohydride. In another preferred embodiment, in Step (f), the solvent used in the reduction is selected from water, methanol, ethanol, or a combination thereof. In another preferred embodiment, the reduction system further contains an inorganic base, and the inorganic base is selected from NaOH, KOH, LiOH, or a combination thereof, and more preferably, NaOH.

In another preferred embodiment, the method for preparing bempedoic acid further includes Step (d): reacting Compound 5 with iso-butyrate to obtain Compound 6. The reaction scheme is as follows: 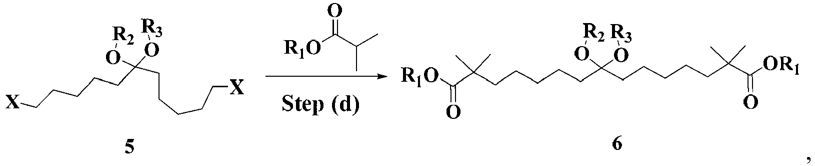 in which X is selected from Cl, Br or I.

In another preferred embodiment, the reaction in Step (d) is carried out in the presence of a base, and the base is selected from lithium diisopropylamide, sodium bis(trimethylsilyl)amide, potassium bis(trimethylsilyl)amide, lithium bis(trimethylsilyl)amide, or a combination thereof, and more preferably, lithium diisopropylamide. In another preferred embodiment, the iso-butyrate is selected from methyl isobutyrate, ethyl isobutyrate, n-propyl isobutyrate, iso-propyl isobutyrate, n-butyl isobutyrate, t-butyl isobutyrate or iso-butyl isobutyrate, and more preferably, methyl isobutyrate or ethyl isobutyrate.

In another preferred embodiment, the reaction in Step (d) is carried out in a solvent-free system. In another preferred embodiment, the reaction in Step (d) is carried out in an aprotic solvent, and the aprotic solvent is selected from tetrahydrofuran, 2-methyltetrahydrofuran, methyl tert-butyl ether, toluene, or a combination thereof, and more preferably, tetrahydrofuran. In another preferred embodiment, in Step (d), the reaction temperature is -30 to 50°C, and more preferably 0 to 30°C.

In another preferred embodiment, the reaction in Step (d) is carried out in the presence of a lithium reagent stabilizer, and the lithium reagent stabilizer is selected from DMPU, HMPA, N,N-dimethyl ethylene diamine, or a combination thereof, and more preferably DMPU.

In another preferred embodiment, the method for preparing bempedoic acid further includes Step (c): reacting Compound 4 with an alcohol to produce Compound 5. The reaction scheme is as follows:

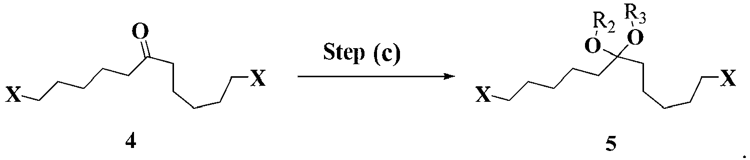

In another preferred embodiment, in Step (c), the reaction of Compound 4 with the alcohol is carried out in the presence of an acid, and the acid is selected from p-toluenesulfonic acid, benzenesulfonic acid, methansulfonic acid, sulfosalicylic acid, naphthalensulfonic acid, trifluoroacetic acid, or a combination thereof, and more preferably, p-toluenesulfonic acid. In another preferred embodiment, the alcohol is selected from methanol, ethanol, propanol, ethylene glycol, 1,3-propylene glycol or 1,2-propylene glycol, and more preferably, ethylene glycol. In another preferred embodiment, in Step (c), the solvent used in the reaction is selected from cyclohexane, toluene, ethylene glycol dimethyl ether, or a combination thereof. In another preferred embodiment, the reaction temperature in Step (c) is 60 to 150°C, and more preferably 80 to 120°C.

In another preferred embodiment, the method for preparing bempedoic acid further includes Step (b): reacting Compound 2 with a halogenating agent to obtain Compound 4. The reaction scheme is as follows:

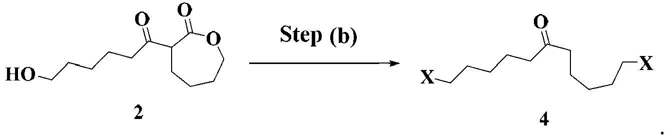

In another preferred embodiment, in Step (b), the halogenating agent is selected from thionyl chloride, hydrogen bromide, phosphorus tribromide or iodine, and more preferably, hydrogen bromide. In another preferred embodiment, the reaction temperature in Step (b) is 45 to 100°C, and more preferably, 60 to 100°C. In another preferred embodiment, in Step (b), the solvent used in the reaction is selected from acetic acid, water, and toluene.

In another preferred embodiment, the method for preparing bempedoic acid further includes the following steps:
(b') hydrolyzing Compound 2 to form Compound 3, and
(b") reacting Compound 3 with a halogenating agent to obtain Compound 4. The reaction scheme is as follows:

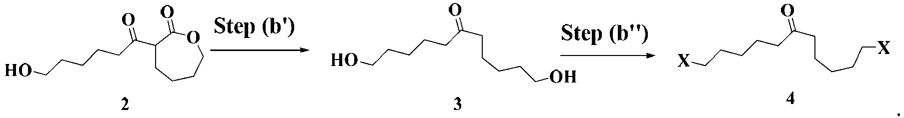

In another preferred embodiment, in Step (b'), the hydrolysis is carried out under a basic Conditions, and the base used in the hydrolysis is selected from KOH, NaOH, LiOH, or a combination thereof, and more preferably NaOH. In another preferred embodiment, the reaction temperature in Step (b') is 50 to 90°C, and more preferably, 60 to 80°C. In another preferred embodiment, the solvent used in the hydrolysis in Step (b') is selected from methanol, ethanol, propanol, water, or a combination thereof.

In another preferred embodiment, in Step (b"), the halogenating agent is selected from thionyl chloride, hydrogen bromide, phosphorus tribromide or iodine. In another preferred embodiment, the halogenation temperature in Step (b") is 45 to 100°C, and more preferably, 60 to 100°C. In another preferred embodiment, the solvent used in the reaction in Step (b") is selected from acetic acid, water, and toluene.

In another preferred embodiment, the method for preparing bempedoic acid further includes Step (a): subjecting Compound 1 to self-condensation to obtain Compound 2. The reaction scheme is as follows:

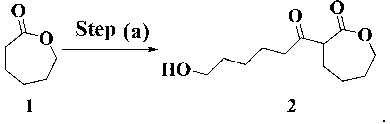

In another preferred embodiment, in Step (a), the self-condensation of Compound 1 is carried out in the presence of titanium tetrachloride and a base, and the base is selected from triethyl amine, tributyl amine, diisopropylethyl amine, or a combination thereof, and more preferably triethyl amine. In another preferred embodiment, in Step (a), the solvent used in the reaction is selected from dichloromethane, toluene, and chloroform, and preferably dichloromethane. In another preferred embodiment, in Step (a), the reaction temperature is - 80 to 50°C, and more preferably, -20 to 30°C.

In another preferred embodiment, the method for preparing bempedoic acid includes the following steps:
(d) reacting Compound 5 with an iso-butyrate to obtain Compound 6, and
(e) hydrolyzing Compound 6, and removing the carbonyl protecting group to obtain Compound 7.

The reaction scheme is as follows: 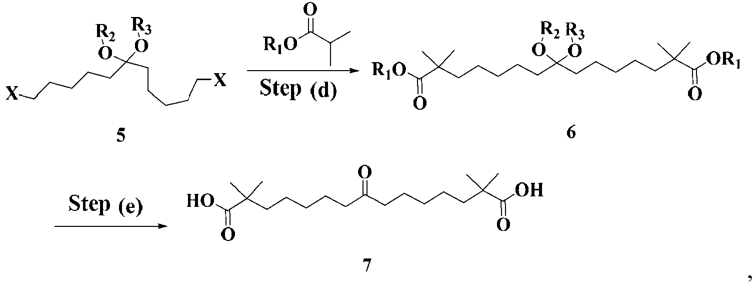 in which X is selected from Cl, Br or I; R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 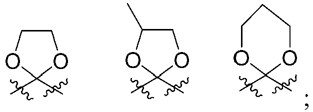 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, X is selected from Br, R₁ is selected from methyl or ethyl, and R₂ and R₃, together with the oxygen and carbon to which they are attached, form

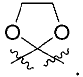

In another preferred embodiment, the method for preparing bempedoic acid includes the following steps:
(c) reacting Compound 4 with an alcohol to produce Compound 5,
(d) reacting Compound 5 with an iso-butyrate to obtain Compound 6, and
(e) hydrolyzing Compound 6 and removing the carbonyl protecting group to obtain Compound 7.

The reaction scheme is as follows: 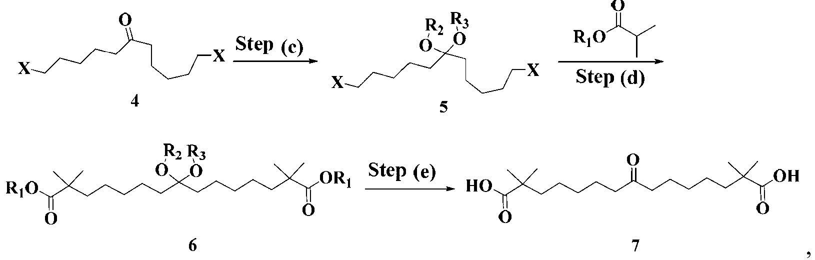 in which X is selected from Cl, Br or I; R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 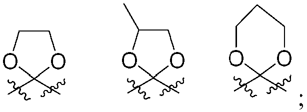 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, X is selected from Br, R₁ is selected from methyl or ethyl, and R₂ and R₃, together with the oxygen and carbon to which they are attached, form

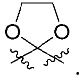

In another preferred embodiment, the method for preparing bempedoic acid includes the following steps:
(b) reacting Compound 2 with a halogenating agent to obtain Compound 4,
(c) reacting Compound 4 with an alcohol to produce Compound 5,
(d) reacting Compound 5 with an iso-butyrate to obtain Compound 6,
(e) hydrolyzing Compound 6, and removing the carbonyl protecting group to obtain Compound 7; and
(f) reducing Compound 7 to obtain bempedoic acid.

The reaction scheme is as follows: 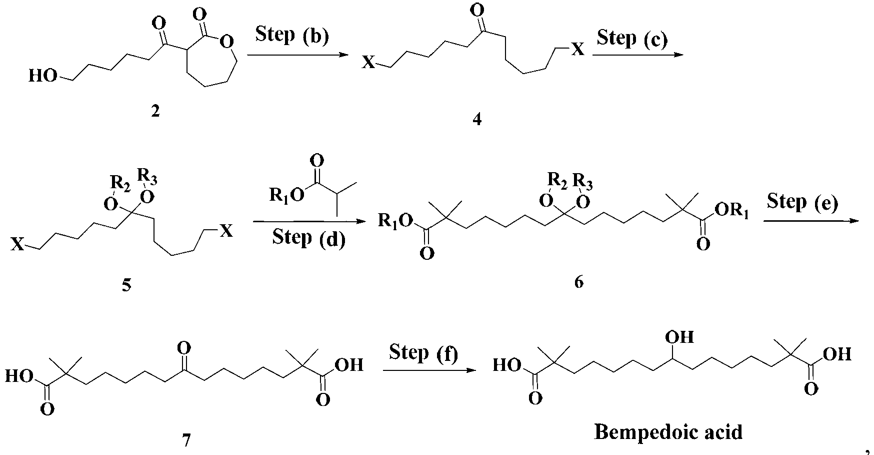 in which X is selected from Cl, Br or I; R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 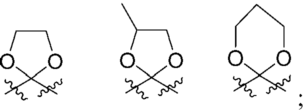 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, X is selected from Br, R₁ is selected from methyl or ethyl, and R₂ and R₃, together with the oxygen and carbon to which they are attached, form

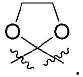

In another preferred embodiment, the method for preparing bempedoic acid includes the following steps:
(b') hydrolyzing Compound 2 to form Compound 3,
(b") reacting Compound 3 with a halogenating agent to obtain Compound 4,
(c) reacting Compound 4 with an alcohol to produce Compound 5,
(d) reacting Compound 5 with an iso-butyrate to obtain Compound 6,
(e) hydrolyzing Compound 6, and removing the carbonyl protecting group to obtain Compound 7; and
(f) reducing Compound 7 to obtain bempedoic acid.

The reaction scheme is as follows: 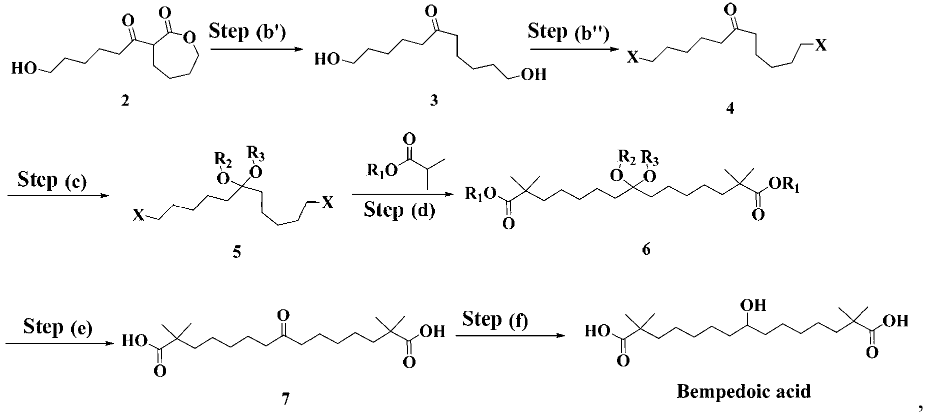 in which X is selected from Cl, Br or I; R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 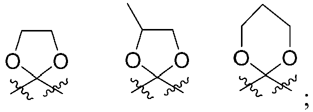 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, X is selected from Br, R₁ is selected from methyl or ethyl, and R₂ and R₃, together with the oxygen and carbon to which they are attached, form

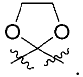

In another preferred embodiment, in the method for preparing bempedoic acid, Compound 2 is prepared through a method including the step of: (a) subjecting Compound 1 to self-condensation to obtain Compound 2

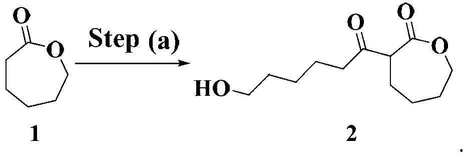

In another aspect, the present application provides a compound having a structure of Formula 5 or 6: 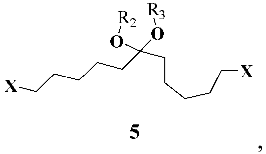 in which X is selected from Cl, Br or I; R₂ and R₃ are each independently selected from a linear or branched C₁-C₆alkyl group, or R₂ and R₃, together with the oxygen and carbon to which they are attached, form 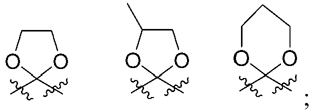 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and 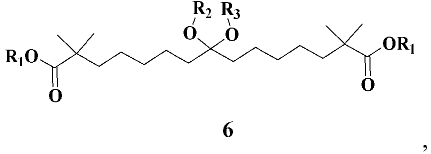 in which R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 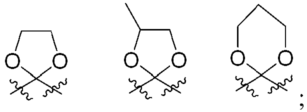 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

In another preferred embodiment, In the compound of Formula 5, X is selected from Br, and R₂ and R₃, together with the oxygen and carbon to which they are attached, form

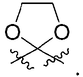

In another preferred embodiment, in the compound of Formula 6, R₁ is selected from methyl or ethyl, and R₂ and R₃, together with the oxygen and carbon to which they are attached, form

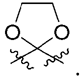

In another aspect, the present invention provides a method for preparing Compound 5, which includes the following steps:
(b) reacting Compound 2 with a halogenating agent to obtain Compound 4, and
(c) reacting Compound 4 with an alcohol to produce Compound 5.

The reaction scheme is as follows:

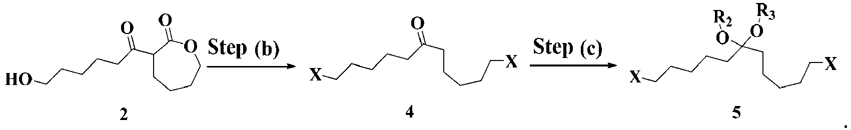

In another aspect, the present invention provides a method for preparing Compound 5, which includes the following steps:
(b') hydrolyzing Compound 2 to form Compound 3,
(b") reacting Compound 3 with a halogenating agent to obtain Compound 4, and
(c) reacting Compound 4 with an alcohol to produce Compound 5.

The reaction scheme is as follows:

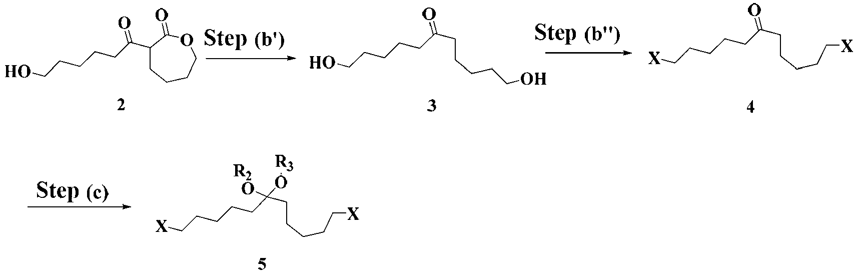

In another aspect, the present invention provides a method for preparing Compound 6, which includes the following steps:
(b) reacting Compound 2 with a halogenating agent to obtain Compound 4,
(c) reacting Compound 4 with an alcohol to produce Compound 5, and
(d) reacting Compound 5 with an iso-butyrate to obtain Compound 6.

The reaction scheme is as follows:

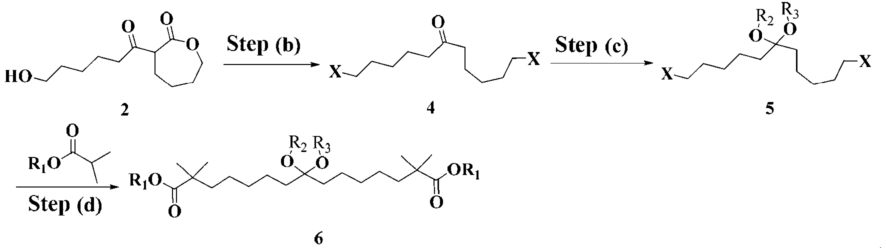

In another aspect, the present invention provides a method for preparing Compound 6, which includes the following steps:
(b') hydrolyzing Compound 2 to form Compound 3,
(b") reacting Compound 3 with a halogenating agent to obtain Compound 4,
(c) reacting Compound 4 with an alcohol to produce Compound 5, and
(d) reacting Compound 5 with an iso-butyrate to obtain Compound 6.

The reaction scheme is as follows:

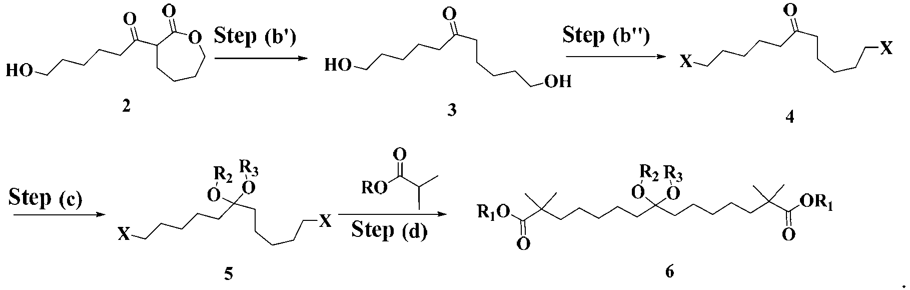

In another aspect, the present invention provides use of Compound 5 and Compound 6 in the preparation of bempedoic acid.

In another aspect, the present invention provides a method for preparing Compound 4, which includes the following steps:
(a) subjecting Compound 1 to self-condensation to obtain Compound 2, and
(b) reacting Compound 2 with a halogenating agent to obtain Compound 4.

The reaction scheme is as follows: 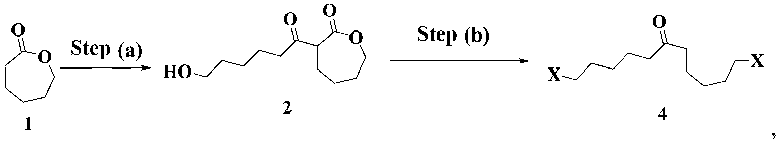 in which X is selected from Cl, Br or I, and more preferably Br.

In another preferred embodiment, the method for preparing Compound 4 provided in the present invention includes the following steps:
(a) subjecting Compound 1 to self-condensation to obtain Compound 2,
(b') hydrolyzing Compound 2 to form Compound 3, and
(b") reacting Compound 3 with a halogenating agent to obtain Compound 4.

The reaction scheme is as follows: 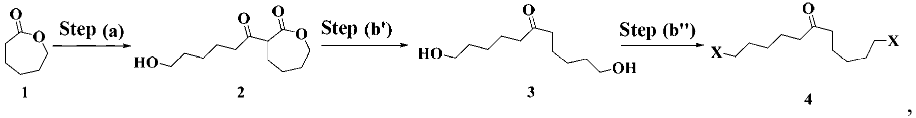 in which X is selected from Cl, Br or I, and more preferably Br.

It is to be understood that within the scope of the present invention, the technical features described above and the technical features specifically described below (in the embodiments) in the present invention may be combined with each other to constitute a new or preferred technical solution. For the sake of brevity, these combinations are not described here.

### BRIEF DESCRIPTION OF THE DRAWINGS

The following drawings are used to illustrate specific embodiments of the invention, and not intended to limit the scope of the present invention defined by the claims.

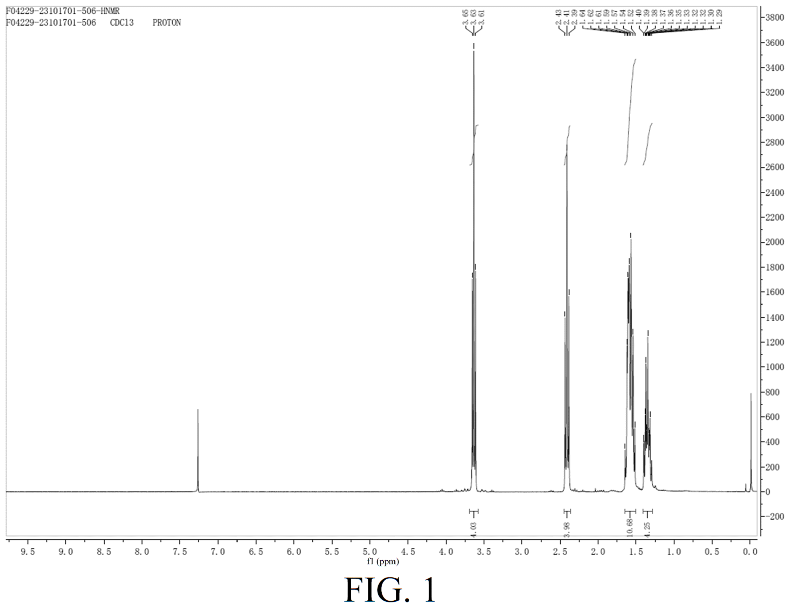

Fig. 1 is a ¹H NMR spectrum of Compound 3 (1,11-dihydroxyundecan-6-one);

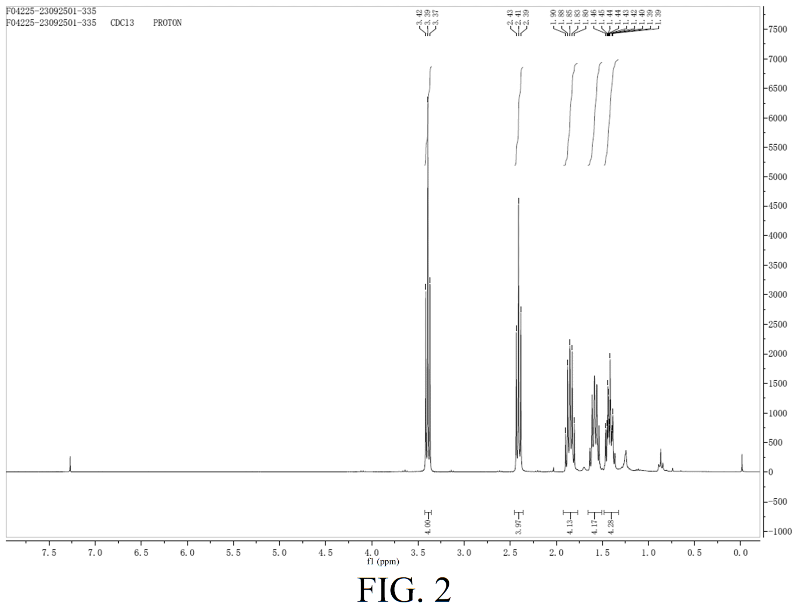

Fig. 2 is a ¹H NMR spectrum of Compound 4 (1,11-dibromoundecan-6-one);

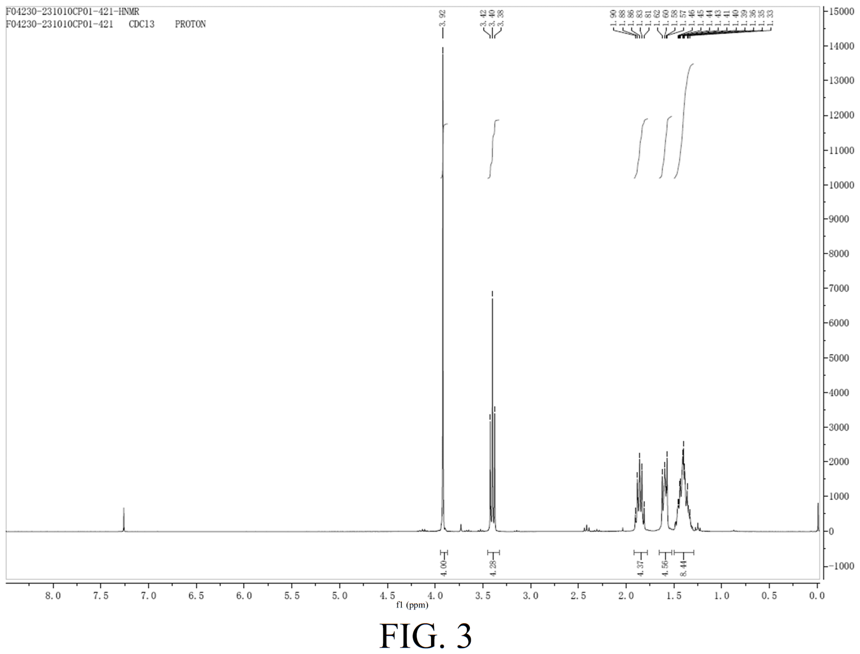

Fig. 3 is a ¹H NMR spectrum of Compound 5 (2,2-bis(5-bromopentyl)-1,3-dioxolane); and

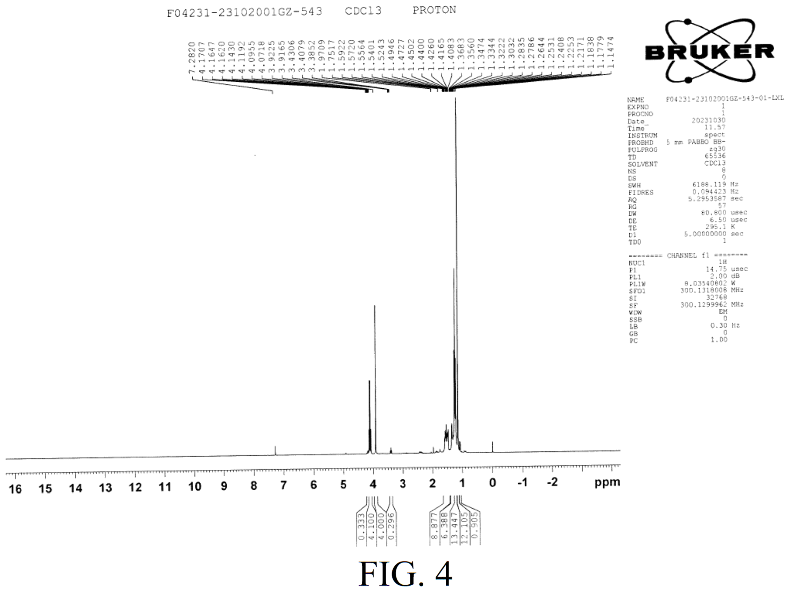

Fig. 4 is a ¹H NMR spectrum of Compound 6 (diethyl 7,7'-(1,3-dioxolan-2,2-diyl)bis(2,2-dimethylheptanoate)).

### DETAILED DESCRIPTION

After extensive and in-depth research, a new method for preparing bempedoic acid is developed. In the method, ε-caprolactone is used as a raw material, which is subjected to self-condensation, hydrolysis, bromination, carbonyl protection, and esterification to obtain new Intermediate 6. The intermediate is hydrolyzed, removed of the hydroxyl protecting group, and reduced to obtain bempedoic acid. Through the method, the problems of high cost, low yield and poor product quality associated with the existing chemical synthesis method of bempedoic acid are solved.

### Preparation of Compound 2

In the present invention, caprolactone is used as a starting material, and the preparation is carried out following the method described in Example 7 of prior art WO2023/147657A1.

### Preparation of Compound 4

In the present invention, with Compound 2 as a raw material, Compound 4 is prepared by the step of:
(b) reacting Compound 2 with a halogenating agent to obtain Compound 4.

The reaction scheme is as follows: 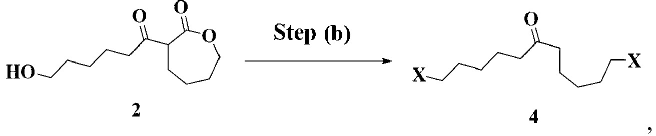 in which X is selected from Cl, Br or I.

In the present invention, with Compound 2 as a raw material, Compound 4 can also be prepared by the step of:
(b') hydrolyzing Compound 2 to form Compound 3, and
(b") reacting Compound 3 with a halogenating agent to obtain Compound 4.

The reaction scheme is as follows:

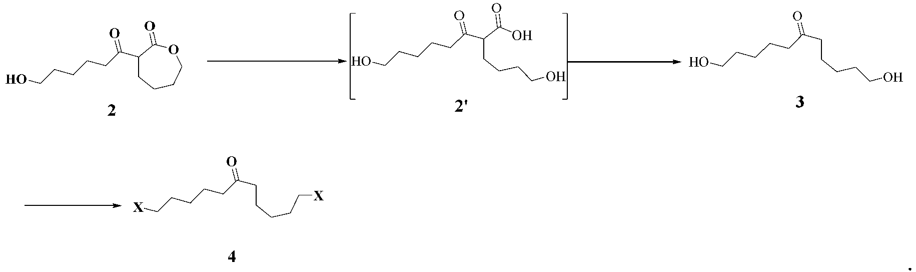

In Step (b) and Step (b"), the halogenating agent includes, but is not limited to, hydrogen chloride, thionyl chloride, hydrogen bromide, phosphorus tribromide, hydrogen iodide, and iodine. The halogenating agent is used in an amount conventionally used for such reactions in the art. Preferably, the molar ratio of the halogenating agent to Compound 2 is 1 to 10:1, and more preferably 4 to 6:1. The solvent used in the halogenation reaction includes, but is not limited to, acetic acid, water, and toluene. In a specific embodiment of the present invention, the halogenation reaction is carried out in an acetic acid solution of hydrogen bromide, and the reaction temperature is preferably 45-100°C, and more preferably, 50 to 80°C.

In Step (b'), the hydrolysis of Compound 2 is carried out under a basic condition. In the process of hydrolysis to form compound 3, Compound 2 is hydrolyzed in the presence of a base, to form intermediate Compound 2', and then Compound 2' is heated (for example, 50 to 90°C, and more preferably 60 to 80°C) to remove the carboxyl group ortho to the carbonyl group, to produce Compound 3. The base used in this step is preferably an inorganic nucleophilic strong base, including, but not limited to, KOH, NaOH, and LiOH. The solvent used in the reaction of Step (b') is a solvent commonly used for such reactions in the art, including but not limited to methanol, ethanol, propanol, and water, etc.

### Preparation of Compound 5

In the present invention, with Compound 4 as a raw material, Compound 5 is prepared by the step of:
Step (c): reacting Compound 4 with an alcohol to produce Compound 5. The reaction scheme is as follows: 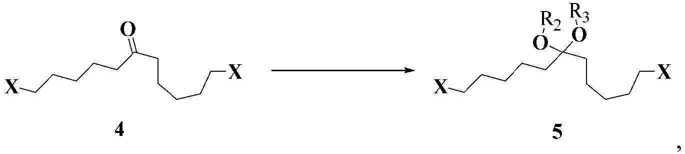
in which X is selected from Cl, Br or I; and R₂ and R₃ are each independently selected from a linear or branched C₁-C₆alkyl group, or R₂ and R₃, together with the oxygen and carbon to which they are attached, form 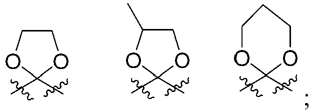
where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

The reaction in this step is carried out in the presence of an acid, which acts as a catalyst in this step. The acid that can be used in this step and its amount are those commonly used for such reactions in the art. The acid includes, but is not limited to, p-toluenesulfonic acid, benzenesulfonic acid, methansulfonic acid, sulfosalicylic acid, naphthalensulfonic acid, and trifluoroacetic acid. The amount of the acid is a catalytic amount. Preferably, the molar ratio of the acid to Compound 4 is preferably 0.01 to 0.1:1. The alcohol is used to form a ketal with the carbonyl group of Compound 4. The alcohol includes, but is not limited to, methanol, ethanol, propanol, ethylene glycol, 1,3-propylene glycol, or 1,2-propylene glycol. The molar ratio of the alcohol to Compound 4 is preferably 2 to 10:1,and more preferably 3 to 7:1. The reaction solvent that can be used in this step is a solvent commonly used for such reactions in the art, including, but not limited to, cyclohexane, toluene, and ethylene glycol dimethyl ether. The reaction temperature in this step is a conventional temperature for such reactions in the art, for example, 70 to 120°C.

### Preparation of Compound 6

In Step (d), with Compound 5 as a raw material, Compound 6 can be prepared through the following steps:
reacting Compound 5 with iso-butyrate to obtain Compound 6. The reaction scheme is as follows: 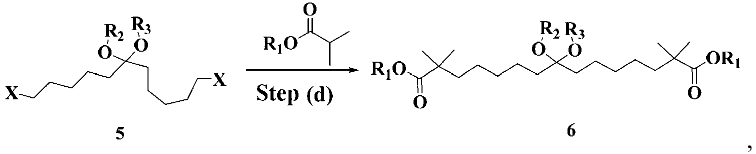
in which X is selected from Cl, Br or I; R₁ is selected from a linear or branched C₁-C₆ alkyl group, a C₁-C₆alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 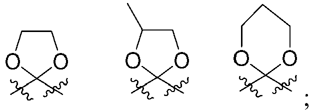 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

This step can be carried out in an aprotic solvent, and the aprotic solvent includes, but is not limited to, tetrahydrofuran, 2-methyltetrahydrofuran, and methyl tert-butyl ether. This step can also be carried out in a solvent-free system (i.e., no solvent is used). The iso-butyrate acts as a solvent where no solvent is used. The reaction in this step is carried out in the presence of a base, and the base is an organic non-nucleophilic strong base, including, but not limited to, lithium diisopropylamide, sodium bis(trimethylsilyl)amide, potassium bis(trimethylsilyl)amide, and lithium bis(trimethylsilyl)amide, etc. The base serves to remove H at α position of isobutyrate to form a carbanion, which has nucleophilicity and, can be nucleophilically substituted for Compound 5, to obtain Compound 6. The base is used in an amount conventionally used for such reactions in the art. For example, the molar ratio of the base to iso-butyrate is 1 to 1.5:1. The molar ratio of iso-butyrate to Compound 4 is preferably 2 to 5:1. The reaction temperature in this step is preferably room temperature. The feeding process in this step is carried out at -20 to -5°C. First, the base is added to the solvent, and then iso-butyrate is added dropwise and stirred for 20 to 40 min, and then Compound 5 was added. After the reaction in this step is completed, a terminating reagent is added to the reaction solution, and the terminating reagent is preferably water, saturated ammonium chloride or dilute hydrochloric acid, and more preferably water.

### Preparation of bempedoic acid

With Compound 6 as a raw material, bempedoic acid is prepared by the steps of:
(e) hydrolyzing Compound 6, and removing the hydroxyl protecting group to obtain Compound 7; and
(f) reducing Compound 7 to obtain bempedoic acid. The reaction scheme is as follows:

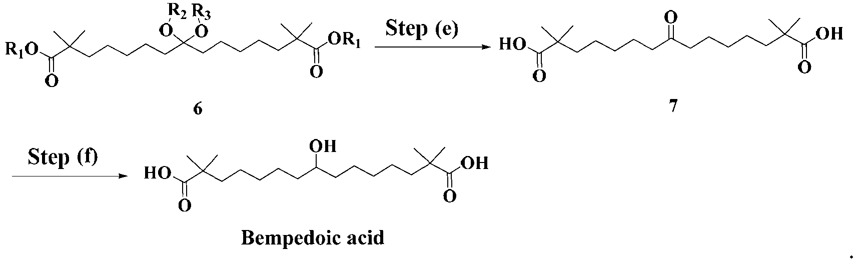

In Step (e), the hydrolysis of Compound 6 can be carried out under a basic condition, or under other conditions. Hydrolysis under a basic condition can be carried out following the conventional operation for such reactions in the art. The base used for hydrolysis includes, but is not limited to, NaOH, KOH, LiOH, Ba(OH)₂, and Me₃SnOH. The solvent used for hydrolysis includes, but is not limited to, water, methanol, ethanol, and propanol. The hydrolysis temperature is 20 to 120°C. The amount of the base is a conventional amount for such reactions. The molar ratio of the base to Compound 6 is preferably 3 to 10:1. The amount of the solvent is a conventional amount for such reactions, and the volume/weight ratio of the solvent to Compound 6 is preferably 5 to 50 mL/g.

In Step (e), the removal of the hydroxyl protecting group under an acidic condition can be carried out following the conventional operation for such reactions in the art. The removal of the carbonyl protecting group is carried out under an acidic condition, and the acid used includes, but is not limited to, hydrochloric acid, sulfuric acid, phosphoric acid, hydrobromic acid, and other inorganic acids. The solvent includes but is not limited to, water, methanol, ethanol, and propanol. The removal of the hydroxyl protecting group is carried out at room temperature. In some specific embodiments, the reaction for removing the hydroxyl protecting group is carried out in a hydrochloric acid aqueous solution with a pH of 1-2 for 2-4 hrs. In some specific embodiments, Compound 6 is hydrolyzed under a basic condition, and the hydroxyl protecting group is directly removed under an acidic condition from a crude product obtained by treating the hydrolyzed solution to obtain Compound 7.

In Step (f), the reduction of Compound 7 to obtain bempedoic acid can be carried out according to the method disclosed in the prior art, for example, WO2004067489, and CN114907204.

The reducing agent useful in the present invention includes, but is not limited to, sodium borohydride, potassium borohydride, lithium borohydride, sodium cyanoborohydride, or sodium triacetoxyborohydride. The solvent used in the reduction reaction includes, but is not limited to, water, methanol, ethanol, and propanol. In some specific embodiments, Compound 7 is reduced with sodium borohydride as a reducing agent in a basic system. The amount of sodium borohydride is a conventional amount in the art, and the molar ratio of sodium borohydride to Compound 7 is preferably 0.9 to 1.1:1. The molar ratio of the base to Compound 7 is 2 to 3:1.

In the reaction process of the present invention, the disappearance of raw materials or the not decreasing of raw materials for a period of time can be detected by a method commonly used in the art (for example, thin-layer chromatography or liquid chromatography) in each step to determine the completion of the reaction in this step and then stop the reaction.

As used herein, the term "room temperature" or "normal temperature" refers to a temperature of 4-40°C, and preferably, 25±5°C.

The term "linear or branched C₁-C₆ alkyl group" refers to a linear or branched hydrocarbon group having a specified number of carbon atoms (i.e., C₁-C₆ represents 1-6 carbon atoms). Examples of alkyl include, but are not limited to, methyl, ethyl, n-propyl, isopropyl, or n-butyl, t-butyl, isobutyl, s-butyl, n-pentyl, n-hexyl and the like.

The term "alkenyl" refers to an unsaturated alkyl group having one or more double bonds. Similarly, the term "alkynyl" refers to an unsaturated alkyl group having one or more triple bonds.

In 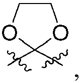 " 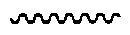 " represents a position of attachment of the formed ketal to the backbone.

The present invention mainly has the following advantages.

According to the preparation method of bempedoic acid in the present invention, caprolactone is used as a starting material, which is subjected to self-condensation, hydrolysis, halogenation, carbonyl protection and α alkylation to obtain novel intermediate Compound 6. Compound 6 is hydrolyzed, deprotected, and reduced, to obtain the target product. The method has the advantages of simple preparation process, short route, high yield, readily available and cheap reagents, good repeatability, good product quality, high purity, and low production cost, thus being applicable to industrial production.

The present invention is further described below in conjunction with specific examples. It is to be understood that these examples are merely illustrative of the present invention and are not intended to limit the scope of the present invention. For experimental methods where no specific conditions are given in the following examples, conventional conditions or conditions recommended by the manufacturer are followed. Unless otherwise specified, percentages and parts are percentages and parts by weight. The reagents and raw materials used in the following examples are generally commercially available unless otherwise specified.

"Purity" mentioned in the following examples refers to HPLC purity.

### Example 1: Preparation of 3-(6-hydroxyhexanoyl)oxacyclopropan-2-one (Compound 2)

At -20°C, ε-caprolactone (150 g, 1.31 mol) and triethyl amine (199.6 g, 1.97 mol) were added to dichloromethane (1.5 L), and then titanium tetrachloride (124.2 g, 0.655 mol) was added, and reacted for 3 hrs while the temperature was maintained. After the reaction was completed, a dilute hydrochloric acid aqueous solution was added (150 ml of concentrated hydrochloric acid added to 450 ml of water). The organic layer was separated, and the aqueous phase was extracted twice with dichloromethane (450 ml x 2). The organic phases were combined, washed once with water (450 ml) and saturated brine (450 ml) respectively, dried over magnesium sulfate, and concentrated under reduced pressure to produce 3-(6-hydroxyhexanoyl)oxacyclopropan-2-one as a light yellow oil (139 g, yield 92.7%, purity 91%). ESI-MS(m/z): [M+H]⁺=227.1

Other reaction conditions in the steps for preparing Compound 2 were studied, and specifically as shown in other examples listed in Table 1 below.

**Table 1**

| | **Base type** | **Base amount** | **Amount of titanium tetrachloride** | **Solvent** | **Reaction temperature** | **Conversion rate** |
|---|---|---|---|---|---|---|
| Example 2 | Triethyl amine | 1.0eq | 0.5 | Dichloromethane | -15°C | 92.1% |
| Example 3 | Triethyl amine | 0.5eq | 0.5 | Dichloromethane | -15°C | 90.6% |
| Example 4 | Triethyl amine | 1.5eq | 1.1 | Dichloromethane | -15°C | 92.3% |
| Example 5 | Triethyl amine | 1.5eq | 0.75 | Dichloromethane | -15°C | 92.0% |
| Example 6 | Triethyl amine | 1.5eq | 0.5 | Dichloromethane | 0°C | 92.3% |
| Example 7 | Triethyl amine | 1.5eq | 0.5 | Dichloromethane | -10°C | 92.5% |
| Example 8 | Tributyl amine | 1.5eq | 0.5 | Dichloromethane | -15°C | 88.3% |
| Example 9 | Diisopropylethyl amine | 1.5eq | 0.5 | Dichloromethane | -15°C | 85.7% |
| Example 10 | Toluene | 1.5eq | 0.5 | Dichloromethane | -15°C | 89.6% |
| Example 11 | Chloroform | 1.5eq | 0.5 | Dichloromethane | -15°C | 90.4% |

### Example 12: Preparation of 1,11-dibromoundecan-6-one (Compound 4)

At room temperature, 3-(6-hydroxyhexanoyl)oxacyclopropan-2-one (138 g, 0.605 mol) was added to a hydrobromic acid/acetic acid solution (109 g, 2.42 mol), heated to 75°C, and reacted for 6 hrs. After the reaction was completed, the reaction solution was cooled to room temperature, added with methanol (690 ml), stirred overnight, and concentrated under reduced pressure. The concentrate was added with dichloromethane (450 ml), dissolved by stirring, extracted twice with water (450 ml x 2), and then washed once with saturated brine (450 ml). The organic phases were combined, dried over magnesium sulfate, and concentrated under reduced pressure to produce 1,1-dibromoundecan-6-one as a light yellow oil (174 g, yield 87.6%, purity 90%). ESI-MS(m/z): [M+H]⁺=329.0. ¹HNMR(300 MHz, CDCl₃): δ3.39(t,*J*=9 Hz, 4H),2.41(t,J=6 Hz, 4H)1.90-1.80(m, 4H),1.64-1.54(m,4H),1.46-1.36(m, 4H).

Other reaction conditions in the steps for preparing Compound 3 were studied, and specifically as shown in other examples listed in Table 2 below.

**Table 2**

| Examples | Acid type | Acid amount | Reaction temperature | Conversion rate |
|---|---|---|---|---|
| Example 13 | 40% hydrobromic acid aqueous solution | 4eq | 75°C | 72.1% |
| Example 14 | Phosphorus tribromide | 2eq | 25°C | 64.8% |
| Example 15 | 33% hydrobromic acid/acetic acid solution | 3eq | 75°C | 87.0% |
| Example 16 | 33% hydrobromic acid/acetic acid solution | 5eq | 75°C | 86.9% |
| Example 17 | 33% hydrobromic acid/acetic acid solution | 4eq | 50°C | 68.7% |
| Example 18 | 33% hydrobromic acid/acetic acid solution | 4eq | 80°C | 85.8% |

### Example 19: Preparation of 2,2-bis(5-bromopentyl)-1,3-dioxolane (Compound 5)

At room temperature, 1,11-dibromoundecan-6-one (174 g, 0.53 mol), ethylene glycol (174 ml) and p-toluenesulfonic acid (9.1 g, 0.05 mol) were added to cyclohexane (1.75 L), heated to reflux, and reacted for 6 hrs. After the reaction was completed, the reaction solution was cooled to room temperature, and extracted and washed with a saturated sodium bicarbonate solution (870 ml). The organic phase was further washed with saturated brine (870 ml), dried over magnesium sulfate, and concentrated under reduced pressure to produce 2,2-bis(5-bromopentyl)-1,3-dioxolane as a light yellow oil (187.6 g, yield of 95.0%, purity 95%). ESI-MS(m/z): [M+H]⁺=371.0. ¹HNMR(300 MHz, CDCl₃): δ 3.92(s, 4H),3.40(t,*J*=6 Hz, 4H)1.90-1.81(m, 4H),1.62-1.57(m, 4H),1.46-1.33(m, 4H).

Other reaction conditions in the steps for preparing Compound 5 were studied, and specifically as shown in other examples listed in Table 3 below.

**Table 3**

| Examples | Alcohol type | Alcohol amount | Amount of p-toluenesulfonic acid | Solvent | Solvent amount | Reaction temperature | Conversion rate |
|---|---|---|---|---|---|---|---|
| Example 20 | 1,3-propyle ne glycol | 1.0v/w | 0.1eq | Cyclohexane | 10v/w | 110°C | 85.3% |
| Example 21 | 1,2-propyle ne glycol | 1.0v/w | 0.1eq | Cyclohexane | 10v/w | 110°C | 82.1% |
| Example 22 | Ethylene glycol | 1.5v/w | 0.1eq | Cyclohexane | 10v/w | 110°C | 96.6% |
| Example 23 | Ethylene glycol | 2.0v/w | 0.1eq | Cyclohexane | 10v/w | 110°C | 95.8% |
| Example 24 | Ethylene glycol | 1.0v/w | 0.05eq | Cyclohexane | 10v/w | 110°C | 84.8% |
| Example 25 | Ethylene glycol | 1.0v/w | 0.2eq | Cyclohexane | 10v/w | 110°C | 96.1% |
| Example 26 | Ethylene glycol | 1.0v/w | 0.1eq | Toluene | 10v/w | 110°C | 88.6% |
| Example 27 | Ethylene glycol | 1.0v/w | 0.1eq | Ethylene glycol dimethyl ether | 10v/w | 110°C | 90.2% |
| Example 28 | Ethylene glycol | 1.0v/w | 0.1eq | Cyclohexane | 5v/w | 110°C | 97.6% |
| Example 29 | Ethylene glycol | 1.0v/w | 0.1eq | Cyclohexane | 15v/w | 110°C | 95.4% |
| Example 30 | Ethylene glycol | 1.0v/w | 0.1eq | Cyclohexane | 10v/w | 80°C | 85.3% |
| Example 31 | Ethylene glycol | 1.0v/w | 0.1eq | Cyclohexane | 10v/w | 100°C | 89.3% |
| Example 32 | Ethylene glycol | 2.0v/w | 0.1eq | Trimethyl orthoformate | 0.5v/w | 35°C | 97.2% |

### Example 33: Preparation of dimethyl 7,7'-(1,3-dioxolan -2,2-diyl)bis(2,2-dimethylheptanoate) (Compound 6)

Diisopropyl amine (141.7 g, 1.4 mol) was added to anhydrous tetrahydrofuran (1.87 L), purged with nitrogen, and then cooled to -10°C. n-Butyllithium (559 ml, 1.4 mol) was slowly added, and stirred for 30 min. Then methyl isobutyrate (132.8 g, 1.3 mol) was added dropwise, and stirred for 30 min. Subsequently, 2,2-bis(5-bromopentyl)-1,3-dioxolane (187 g, 0.50mol) was added, heated to 20-30°C, and reacted for 6 hrs. After the reaction was completed, water (950 ml) and then ethyl acetate (1.87L) were added, and stirred. The organic phase was separated, and the aqueous phase was washed with ethyl acetate (2 L), dried over magnesium sulfate, and concentrated under reduced pressure to produce dimethyl 7,7'-(1,3-dioxolan -2,2-yl)bis(2,2-dimethylheptanoate) as a light yellow oil (190.7 g, yield 92%, purity 97%).

ESI-MS(m/z): [M+H]⁺=415.2. ¹HNMR (300 MHz, CDCl₃): δ 3.92(d,*J*=1.80Hz, 4H),1.59-1.32(m, 20H),1.23(t,*J*=4.65 Hz, 6H),1.16(s, 12H).

### Example 34: Preparation of diethyl 7,7'-(1,3-dioxolan-2,2-diyl)bis(2,2-dimethylheptanoate) (Compound 6)

Diisopropyl amine (141.7 g, 1.4mol) was added to anhydrous tetrahydrofuran (1.87 L), purged with nitrogen, and then cooled to -10°C. n-Butyllithium (559 ml, 1.4 mol) was slowly added and stirred for 30 min. Then ethyl isobutyrate (151 g, 1.3 mol) was added dropwise and stirred for 30 min. Subsequently, 2,2-bis(5-bromopentyl)-1,3-dioxolane (187 g, 0.50 mol) was added, heated to 20-30°C, and reacted for 6 hrs. After the reaction was completed, water (950 ml) and then ethyl acetate (1.87 L) were added, and stirred. The organic phase was separated, and the aqueous phase was washed with ethyl acetate (1.87 L), dried over magnesium sulfate, and concentrated under reduced pressure to produce diethyl 7,7'-(1,3-dioxolan-2,2-diyl)bis(2,2-dimethylheptanoate) as a light yellow oil (201.2 g, yield 90%, purity 97%).

ESI-MS(m/z): [M+H]⁺=443.1. ¹HNMR (300 MHz, CDCl₃): δ 4.11(t,*J*=7.14 Hz, 4H), 3.92(d,*J*=1.80 Hz, 4H),1.59-1.32(m, 20H),1.23(t,*J*=4.65 Hz, 6H),1.16(s, 12H).

Other reaction conditions in the steps for preparing Compound 6 were studied, and specifically as shown in other examples listed in Table 4 below.

**Table 4**

| Examples | Base type | Base amount | Ester type | Ratio of ester: base | Solvent used in the reaction | Reaction temperature | Termination reagent | Conversion rate |
|---|---|---|---|---|---|---|---|---|
| Example 35 | Sodium bis(trimethylsilyl)amide | 2.8eq | Ethyl isobutyrate | 1.05 | Tetrahydrofuran | 25°C | Water | 89.3% |
| Example 36 | Potassium bis(trimethylsilyl)amide | 2.8eq | Ethyl isobutyrate | 1.05 | Tetrahydrofuran | 25°C | Water | 88.4% |
| Example 37 | Lithium bis(trimethylsilyl)amide | 2.8eq | Ethyl isobutyrate | 1.05 | Tetrahydrofuran | 25°C | Water | 88.6% |
| Example 38 | Lithium diisopropylamide | 2.0eq | Ethyl isobutyrate | 1.05 | Tetrahydrofuran | 25°C | Water | 74.2% |
| Example 39 | Lithium diisopropylamide | 2.5eq | Ethyl isobutyrate | 1.05 | Tetrahydrofuran | 25°C | Water | 88.7% |
| Example 40 | Lithium diisopropylamide | 2.8eq | Methyl isobutyrate | 1.05 | Tetrahydrofuran | 25°C | Water | 92.6% |
| Example 41 | Lithium diisopropylamide | 2.8eq | Propyl isobutyrate | 1.05 | Tetrahydrofuran | 25°C | Water | 84.6% |
| Example 42 | Lithium diisopropylamide | 2.8eq | Ethyl isobutyrate | 1.00 | Tetrahydrofuran | 25°C | Water | 90.6% |
| Example 43 | Lithium diisopropylamide | 2.8eq | Ethyl isobutyrate | 1.05 | Methyl tert-butyl ether | 25°C | Water | 79.3% |
| Example 44 | Lithium diisopropylamide | 2.8eq | Ethyl isobutyrate | 1.05 | Toluene | 25°C | Water | 81.2% |
| Example 45 | Lithium diisopropylamide/DMPU | 2.8eq/2.5 eq | Ethyl isobutyrate | 1.05 | Tetrahydrofuran | 25°C | Water | 93.5% |
| Example 46 | Lithium diisopropylamide/HMPA | 2.8eq/2.5 eq | Ethyl isobutyrate | 1.05 | Tetrahydrofuran | 25°C | Water | 89.8% |
| Example 47 | Lithium diisopropylamide | 2.8eq | Ethyl isobutyrate | 1.05 | DMF | 25°C | Water | 92.3% |
| Example 48 | Lithium diisopropylamide | 2.8eq | Ethyl isobutyrate | 1.05 | Tetrahydrofuran/ DMF | 25°C | Water | 92.1% |
| Example 49 | Lithium diisopropylamide | 2.8eq | Ethyl isobutyrate | 1.05 | / | 25°C | Water | 78.1% |
| Example 50 | Lithium diisopropylamide | 2.8eq | Ethyl isobutyrate | 1.05 | Tetrahydrofuran | 0°C | Water | 91.5% |
| Example 51 | Lithium diisopropylamide | 2.8eq | Ethyl isobutyrate | 1.05 | Tetrahydrofuran | 30°C | Water | 93.2% |
| Example 52 | Lithium diisopropylamide | 2.8eq | Ethyl isobutyrate | 1.05 | Tetrahydrofuran | 25°C | Saturated NH₄Cl aqueous solution | 92.5% |
| Example 53 | Lithium diisopropylamide | 2.8eq | Ethyl isobutyrate | 1.05 | Tetrahydrofuran | 25°C | Saturated brine | 92.1% |

### Example 54: Preparation of 1,11-dihydroxyundecan-6-one (Compound 3)

At room temperature, 3-(6-hydroxyhexanoyl)oxacyclopropan-2-one (138 g, 0.605 mol) and sodium hydroxide (72.6 g, 1.815 mol) were added to a solution of methanol (500 ml) and water (250 ml), stirred overnight at room temperature, heated to 65°C, and reacted for 3 hrs. After the reaction was completed, the reaction solution was cooled to room temperature, and diluted with water (1.25 L). and methanol was removed by concentration under reduced pressure. The concentrate was adjusted to pH 1 to 2 with concentrated hydrochloric acid (250 ml), and extracted with dichloromethane (500 ml **x 2**). The organic phase was washed once with saturated brine (450 ml). The organic phases were combined, dried over magnesium sulfate, and concentrated under reduced pressure to produce 1,11-dihydroxyundecan-6-one as a light yellow oil (102 g, yield 83.4%, purity 93%). MS: [M+H]⁺=203.1.

ESI-MS(m/z): [M+H]⁺=203.1. ¹HNMR (300 MHz, CDCl₃): δ3.63(t, *J*=6.0Hz, 4H), 2.41 (t, *J*=6.0Hz, 4H,),1.64-1.52(m, 8H),1.40-1.29(m, 4H).

Other reaction conditions in the steps for preparing Compound 3 were studied, and specifically as shown in other examples listed in Table 5 below.

**Table 5**

| Examples | Base type | Base amount | Solvent | Reaction temperature | Conversion rate |
|---|---|---|---|---|---|
| Example 55 | Lithium hydroxide | 3.0eq | Methanol/water | 65°C | 85.6% |
| Example 56 | Potassium hydroxide | 3.0eq | Methanol/water | 65°C | 86.5% |
| Example 57 | Sodium hydroxide | 3.0eq | Methanol | 65°C | 89.2% |
| Example 58 | Sodium hydroxide | 3.0eq | Ethanol | 65°C | 88.4% |
| Example 59 | Sodium hydroxide | 3.0eq | Water | 65°C | 84.1% |
| Example 60 | Sodium hydroxide | 3.0eq | Methanol/water | 60°C | 89.3% |
| Example 61 | Sodium hydroxide | 3.0eq | Methanol/water | 70°C | 90.1% |

### Example 62: Preparation of 1,11-dibromoundecan-6-one (Compound 4)

At room temperature, 1,11-dihydroxyundecan-6-one (102 g, 0.505 mol) was added to a 40% hydrobromic acid aqueous solution (306.4 g, 1.515 mol), heated to reflux and reacted for 6 hrs. After the reaction was completed, the reaction solution was cooled to room temperature, and extracted with dichloromethane (500 ml x 2). The organic phase was washed once with saturated brine (450 ml). The organic phases were combined, dried over magnesium sulfate, and concentrated under reduced pressure to produce 1,11-dibromoundecan-6-one as a light yellow oil (154 g, yield 93.6%, purity 93%). ESI-MS (m/z): [M+H]⁺=329.0. ¹HNMR(300 MHz, CDCl₃): δ 3.39(t,*J*=9 Hz, 4H,),2.41(t,*J*=6 Hz, 4H)1.90-1.80(m, 4H),1.64-1.54(m, 4H),1.46-1.36(m, 4H).

Other reaction conditions in the steps for preparing Compound 4 were studied, and specifically as shown in other examples listed in Table 6 below.

**Table 6**

| Examples | **Acid type** | **Acid amount** | **Reaction temperature** | **Conversion rate** |
|---|---|---|---|---|
| Example 63 | 33% hydrobromic acid/acetic acid solution | 3eq | 75°C | 92.1 % |
| Example 64 | Phosphorus tribromide | 3eq | 25°C | 84.8% |
| Example 65 | 40% hydrobromic acid aqueous solution | 5eq | 75°C | 97.0% |
| Example 66 | 40% hydrobromic acid aqueous solution | 10eq | 75°C | 96.9% |
| Example 67 | 40% hydrobromic acid aqueous solution | 3eq | 50°C | 88.7% |
| Example 68 | 40% hydrobromic acid aqueous solution | 3eq | 80°C | 95.8% |

### Example 69: Preparation of 2,2,14,14-tetramethyl-8-oxopentadecandioic acid (Compound 7)

At room temperature, diethyl 7,7'-(1,3-dioxolan-2,2-diyl)bis(2,2-dimethylheptanoate) (200 g, 0.45 mol) and a 20% sodium hydroxide solution (450ml) were added to ethanol (1 L), heated to reflux and reacted for 3 hrs. After the reaction was completed, the reaction solution was added with water (1 L), cooled to room temperature, and extracted twice with ethyl acetate (500 ml x 2). The aqueous phase was collected, adjusted to pH 1 to 2 with a 2 mol/L hydrochloric acid solution at 20-30°C, and stirred for 3 hrs while the temperature was held. A crude product was obtained after filtration and drying. At room temperature, the crude product was added to methanol (1 L), and stirred until it was completely dissolved. Purified water (200 ml) was added, and stirred for 30 min. Then purified water (400 ml) was further added, stirred for 1 h, filtered, and dried to obtain 2,2,14,14-tetramethyl-8-oxopentadecandioic acid as a light yellow solid (133.1 g, yield 86%, purity 99%).

ESI-MS (m/z): [M+H]⁺=343.2. ¹HNMR (300 MHz, CDCl₃): δ13.00-10.40(br, 2H), 2.38(t,*J*=7.4 Hz, 4H),1.71-1.44(m, 8H),1.37-1.22(m, 8H),1.18(s, 12H).

### Example 70: Preparation of 2,2,14,14-tetramethyl-8-oxopentadecandioic acid (Compound 7)

At room temperature, diethyl 7,7'-(1,3-dioxolan-2,2-diyl)bis(2,2-dimethylheptanoate) (60 g) was added to methanol (300 ml) and a dilute sulfuric acid aqueous solution (6.6 g of concentrated sulfuric acid dissolved in 114 g of water), stirred until uniform, heated to 55 to 65°C, and reacted for 3 hrs while the temperature was maintained. Then, a 30 wt% sodium hydroxide solution (109 g) was added to the reaction system, heated to 75 to 85°C, and further reacted for 4 to 6 hrs. After the reaction was completed, methanol was removed by concentration under reduced pressure. The concentrate was cooled to 0 to 10°C, added with methyl t-butyl ether (300 ml), and adjusted to pH 1 to 2 with concentrated hydrochloric acid at this temperature. The organic layer was separated, and the aqueous phase was extracted once with methyl t-butyl ether (150 ml). The methyl t-butyl ether phases were combined, and washed three times with water (300 ml*3). Methyl tert-ether was reduced to 100 ml by concentration under reduced pressure, n-heptane (600 ml) was added dropwise at 50-60°C, and the temperature was reduced for crystallization. After filtering and drying, 2,2,14,14-tetramethyl-8-oxopentadecandioic acid as an off-white solid was obtained (42.2 g, yield 91%, purity 98.6%).

### Example 71: 2,2,14,14-tetramethyl-8-hydroxypentadecandioic acid (bempedoic acid)

Under a nitrogen atmosphere, sodium hydroxide (33.2 g) was added to purified water (660 g), and dissolved by stirring. Then 2,2,14,14-tetramethyl-8-oxopentadecandioic acid (133 g) was added, and maintained at 20-30°C. Then, sodium borohydride (8.2g)-sodium hydroxide aqueous solution (sodium hydroxide: 0.31 g, purified water: 57.6 g) was added dropwise and reacted for 2 hrs while the temperature was maintained. After the reaction was completed, methyl t-butyl ether (1 kg) was added, and cooled to 0-10°C. Concentrated hydrochloric acid (130 g) was added dropwise, to adjust the solution to pH 1 to 2. After standing, the organic layer was separated, and the organic phase was washed twice with water (500 ml x 2). The concentration was stopped when the organic phase was concentrated under reduced pressure to 150 ml remaining in the reactor. The remaining material in the reacted was heated to 55°C, n-heptane (450 g) was added, and stirred for 30 min while the temperature was held. The reaction solution was cooled to 20°C, and stirred for 3 hrs while the temperature was held. After filtering and drying, BEM was obtained (131 g, 98%; purity 99.5%).

ESI-MS(m/z): [M+H]⁺=345.2. ¹HNMR (300 MHz, DMSO-*d6*): δ11.99(brs, 2H), 4.20(d, *J*=5.3 Hz, 1H),3.35(brs, 1H),1.53-1.12(m, 20H),1.06(s, 12H).

All documents mentioned in the present invention are cited as references in this invention, as if each document is individually cited as a reference. Moreover, it should be understood that after reading the above teachings of the present invention, various changes or modifications can be made by those skilled in the art to the present invention, which also fall within the scope defined by the appended claims of the present invention.

## Claims

1. A method for preparing bempedoic acid, comprising the following steps:
(e) hydrolyzing Compound 6, and removing the carbonyl protecting group to obtain Compound 7, and
(f) reducing Compound 7 to obtain bempedoic acid,
wherein the reaction scheme is as follows: 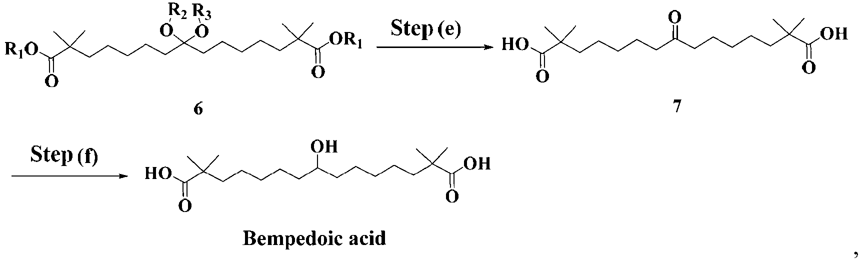
in which R₁ is selected from a linear or branched C₁-C₆ alkyl group, a C₁-C₆ alkenyl group or a C₁-C₆ cycloalkyl group, and R₂ and R₃ are each independently selected from a linear or branched C₁-C₆alkyl group, or R₂ and R₃, together with the oxygen and carbon to which they are attached, form 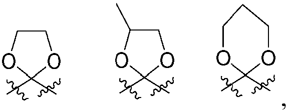 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl.

2. The method for preparing bempedoic acid according to claim 1, wherein R₁ is selected from methyl or ethyl, and R₂ and R₃, together with the oxygen and carbon to which they are attached, form

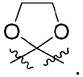

3. The method for preparing bempedoic acid according to claim 1 or 2, wherein in Step (e), the hydrolysis is carried out under a basic condition, and the base is selected from NaOH, KOH, LiOH, Ba(OH)₂, Me₃SnOH or a combination thereof, and more preferably, NaOH; and/or
in Step (e), the removal of the carbonyl protecting group is carried out under an acidic condition, and the acid is selected from hydrochloric acid, sulfuric acid, hydrobromic acid, or a combination thereof, and more preferably, hydrochloric acid; and/or
in Step (e), the carbonyl protecting group is removed from Compound 6, followed by hydrolysis to obtain Compound 7; and and/or
in Step (f), the reducing agent used for the reduction is selected from sodium borohydride, potassium borohydride, lithium borohydride, sodium cyanoborohydride, or sodium triacetoxyborohydride, and more preferably, sodium borohydride.

4. The method for preparing bempedoic acid according to claim 1, further comprising Step (d): subjecting Compound 5 and an iso-butyrate to an α alkylation reaction to obtain Compound 6, wherein the reaction scheme is as follows: 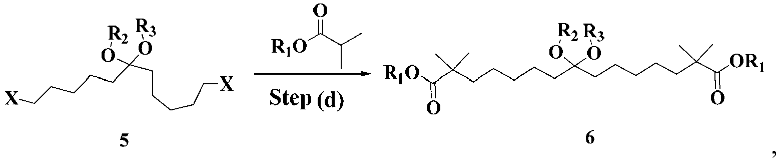 in which X is selected from Cl, Br or I, and more preferably, Br.

5. The method for preparing bempedoic acid according to claim 4, wherein the reaction in Step (d) is carried out in the presence of a base, and the base is selected from lithium diisopropylamide, sodium bis(trimethylsilyl)amide, potassium bis(trimethylsilyl)amide, lithium bis(trimethylsilyl)amide, or a combination thereof, more preferably, lithium diisopropylamide, and more preferably, diisopropyl amine; and/or
the iso-butyrate is selected from methyl isobutyrate, ethyl isobutyrate, n-propyl isobutyrate, iso-propyl isobutyrate, n-butyl isobutyrate, t-butyl isobutyrate or iso-butyl isobutyrate, and more preferably, methyl isobutyrate or ethyl isobutyrate; and/or
the reaction is carried out in a solvent-free system or in an aprotic solvent, and the aprotic solvent is selected from tetrahydrofuran, 2-methyltetrahydrofuran, methyl tert-butyl ether, toluene, or a combination thereof, and more preferably tetrahydrofuran.

6. The method for preparing bempedoic acid according to claim 4, further comprising Step (c): reacting Compound 4 with an alcohol to produce Compound 5, wherein the reaction scheme is as follows:

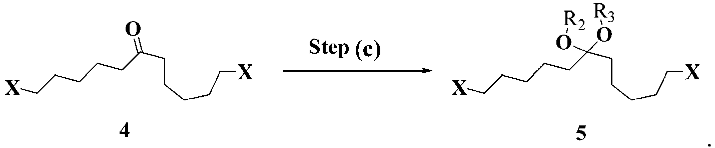

7. The method for preparing bempedoic acid according to claim 6, wherein in Step (c), the reaction of Compound 4 with the alcohol is carried out in the presence of an acid, and the acid is selected from p-toluenesulfonic acid, benzenesulfonic acid, methansulfonic acid, sulfosalicylic acid, naphthalensulfonic acid, trifluoroacetic acid, or a combination thereof, and more preferably, p-toluenesulfonic acid; and/or
the alcohol is selected from methanol, ethanol, propanol, ethylene glycol, 1,3-propylene glycol, or 1,2-propylene glycol.

8. The method for preparing bempedoic acid according to claim 6, further comprising Step (b): reacting Compound 2 with a halogenating agent to obtain Compound 4, wherein the reaction scheme is as follows:

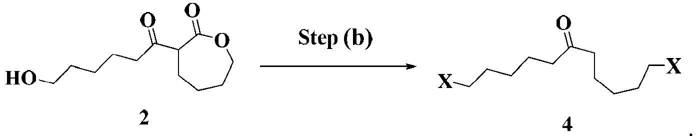

9. The method for preparing bempedoic acid according to claim 6, further comprising the following steps:
(b') hydrolyzing Compound 2 to form Compound 3, and
(b") reacting Compound 3 with the halogenating agent to obtain Compound 4, wherein the reaction scheme is as follows:

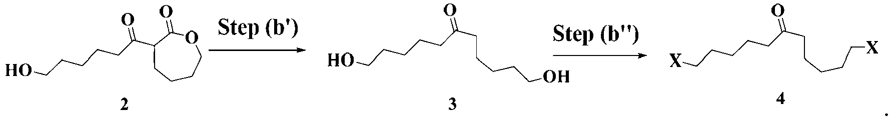

10. The method for preparing bempedoic acid according to claim 8 or 9, wherein the halogenating agent is selected from thionyl chloride, hydrogen bromide, phosphorus tribromide or iodine, and more preferably hydrogen bromide; and/or
the hydrolysis of Compound 2 is carried out under a basic condition, and the base is selected from NaOH, KOH, LiOH, or a combination thereof.

11. The method for preparing bempedoic acid according to claim 8 or 9, further comprising Step (a): subjecting Compound 1 to self-condensation to obtain Compound 2, wherein the reaction scheme is as follows:

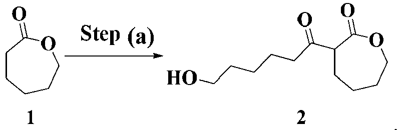

12. The method for preparing bempedoic acid according to claim 11, wherein in Step (a), the condensation is carried out in the presence of titanium tetrachloride and a base, and the base is selected from triethyl amine, tributyl amine, diisopropylethyl amine, or a combination thereof, and more preferably triethyl amine.

13. A method for preparing bempedoic acid, comprising the following steps:
(d): subjecting Compound 5 and an iso-butyrate to an α alkylation reaction to obtain Compound 6, and
(e) hydrolyzing Compound 6, and removing the carbonyl protecting group to obtain Compound 7,
wherein the reaction scheme is as follows: 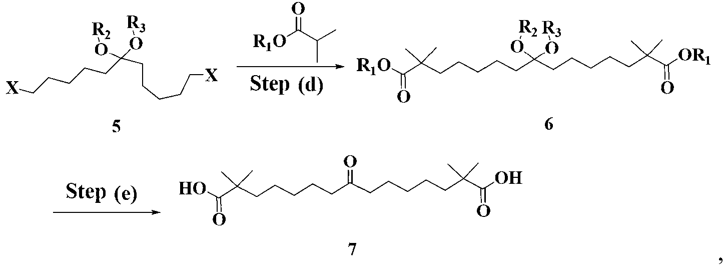
in which X is selected from Cl, Br or I; R₁ is selected from a linear or branched C₁-C₆ alkyl group, a C₁-C₆ alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 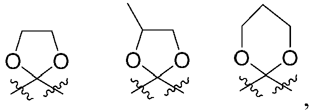 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and more preferably, X is selected from Br, R₁ is selected from methyl or ethyl, and R₂ and R₃, together with the oxygen and carbon to which they are attached, form

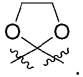

14. A method for preparing bempedoic acid, comprising the following steps:
(c): subjecting Compound 4 and an alcohol to a dehydration reaction to produce Compound 5,
(d): subjecting Compound 5 and an iso-butyrate to an α alkylation reaction to obtain Compound 6, and
(e) hydrolyzing Compound 6 and removing the carbonyl protecting group to obtain Compound 7, wherein the reaction scheme is as follows: 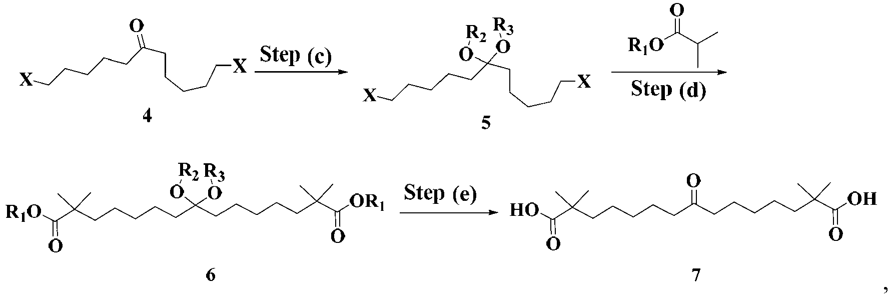
in which X is selected from Cl, Br or I; R₁ is selected from a linear or branched C₁-C₆ alkyl group, a C₁-C₆ alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 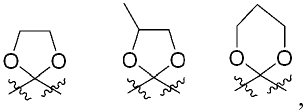 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and more preferably, X is selected from Br, R₁ is selected from methyl or ethyl, and R₂ and R₃, together with the oxygen and carbon to which they are attached, form

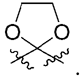

15. A method for preparing bempedoic acid, comprising the following steps:
(b) reacting Compound 2 with a halogenating agent to obtain Compound 4,
(c) reacting Compound 4 with an alcohol to produce Compound 5,
(d) reacting Compound 5 with an iso-butyrate to obtain Compound 6, and
(e) hydrolyzing Compound 6, and removing the carbonyl protecting group to obtain Compound 7, and
(f) reducing Compound 7 to obtain bempedoic acid, wherein the reaction scheme is as follows: 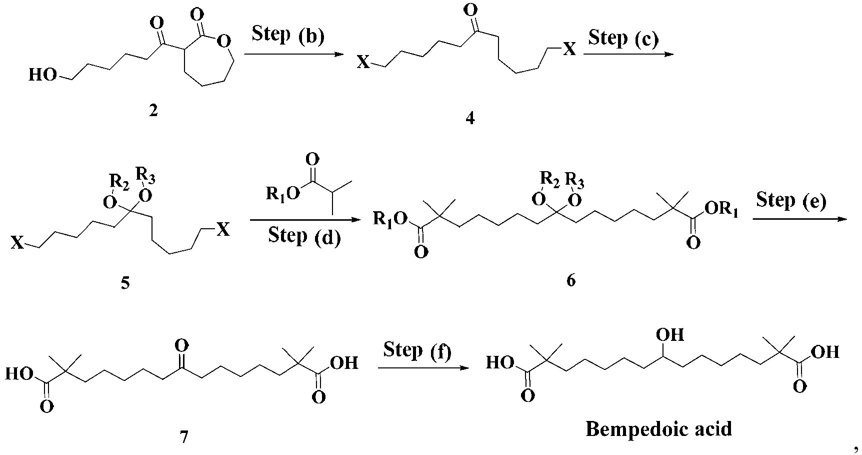
in which X is selected from Cl, Br or I; R₁ is selected from a linear or branched C₁-C₆ alkyl group, a C₁-C₆ alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 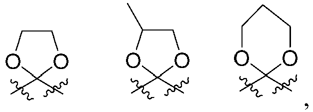 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and more preferably, X is selected from Br, R₁ is selected from methyl or ethyl, and R₂ and R₃, together with the oxygen and carbon to which they are
attached, form

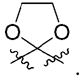

16. A method for preparing bempedoic acid, comprising the following steps:
(b') hydrolyzing Compound 2 to form Compound 3,
(b") reacting Compound 3 with a halogenating agent to obtain Compound 4,
(c) reacting Compound 4 with an alcohol to produce Compound 5,
(d) reacting Compound 5 with an iso-butyrate to obtain Compound 6,
(e) hydrolyzing Compound 6, and removing the carbonyl protecting group to obtain Compound 7, and
(f) reducing Compound 7 to obtain bempedoic acid, wherein the reaction scheme is as follows: 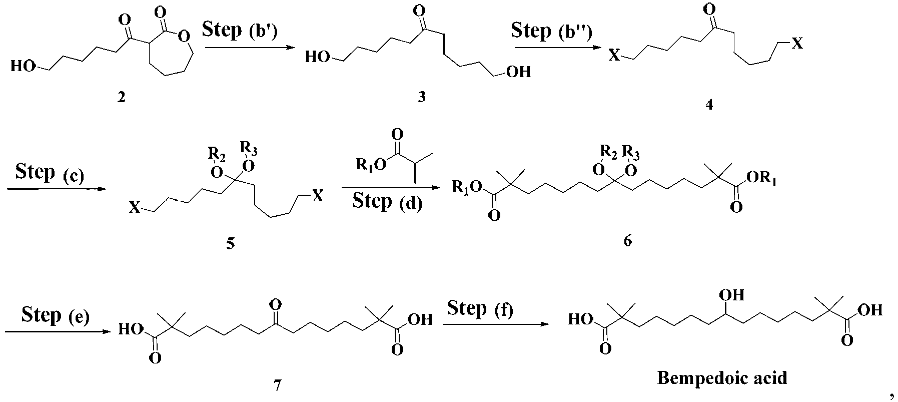
in which X is selected from Cl, Br or I; R₁ is selected from a linear or branched C₁-C₆ alkyl group, a C₁-C₆ alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 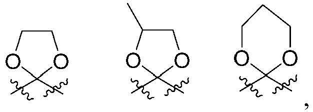 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and more preferably, X is selected from Br, R₁ is selected from methyl or ethyl, and R₂ and R₃, together with the oxygen and carbon to which they are attached, form

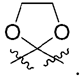

17. The method for preparing bempedoic acid according to claims 15 and 16, wherein Compound 2 is prepared through a method comprising the step of: (a) subjecting Compound 1 to self-condensation to obtain Compound 2

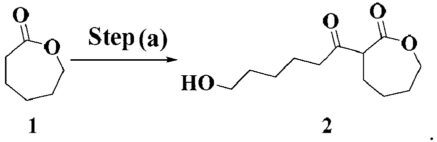

18. A compound, having a structure of Formula 5 or 6: 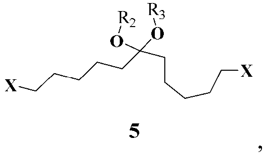 in which X is selected from Cl, Br or I; R₂ and R₃ are each independently selected from H, and a linear or branched C₁-C₆ alkyl group, where the linear or branched C₁-C₆ alkyl group is optionally further substituted with a substituent selected from alkyl, alkoxy, cycloalkyl, and aryl; or R₂ and R₃, together with the oxygen and carbon to which they are attached, form 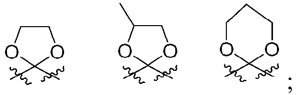 and more preferably, X is selected from Br, R₂ and R₃, together with the oxygen and carbon to which they are attached, form 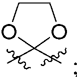 and 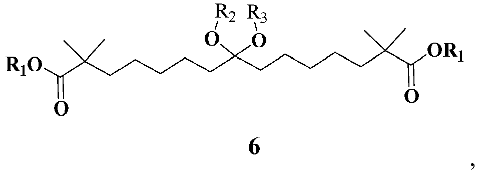 in which R₁ is selected from a linear or branched C₁-C₆ alkyl group, a C₁-C₆ alkenyl group or a C₁-C₆ cycloalkyl group; and R₂ and R₃ are each independently selected from a linear or branched C₁-C₆alkyl group, or R₂ and R₃, together with the oxygen and carbon to which they are attached, form 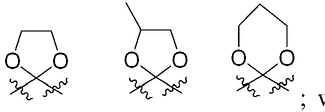 where the linear or branched C₁-C₆ alkyl group, the C₁-C₆ alkenyl group or the C₁-C₆ cycloalkyl group is optionally further substituted with a substituent selected from alkyl, alkoxy, cycloalkyl, and aryl; and more preferably, R₁ is selected from methyl or ethyl, and R₂ and R₃, together with the oxygen and carbon to which they are attached, form

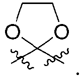

19. A method for preparing Compound 5, comprising the following steps:
(b) reacting Compound 2 with a halogenating agent to obtain Compound 4, and
(c) reacting Compound 4 with an alcohol to produce Compound 5,
wherein the reaction scheme is as follows: 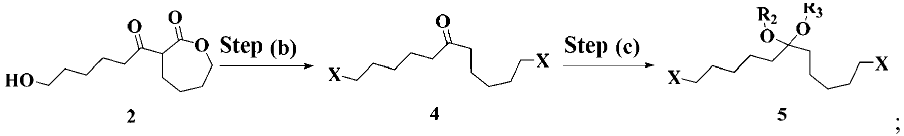
or comprising the following steps:
(b') hydrolyzing Compound 2 to form Compound 3,
(b") reacting Compound 3 with a halogenating agent to obtain Compound 4, and
(c) reacting Compound 4 with an alcohol to produce Compound 5,
wherein the reaction scheme is as follows:

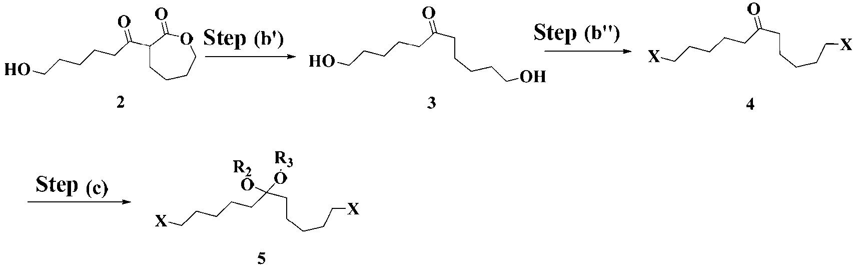

20. A method for preparing Compound 6, comprising the following steps:
(b) reacting Compound 2 with a halogenating agent to obtain Compound 4,
(c) reacting Compound 4 with an alcohol to produce Compound 5, and
(d) reacting Compound 5 with an iso-butyrate to obtain Compound 6,
wherein the reaction scheme is as follows: 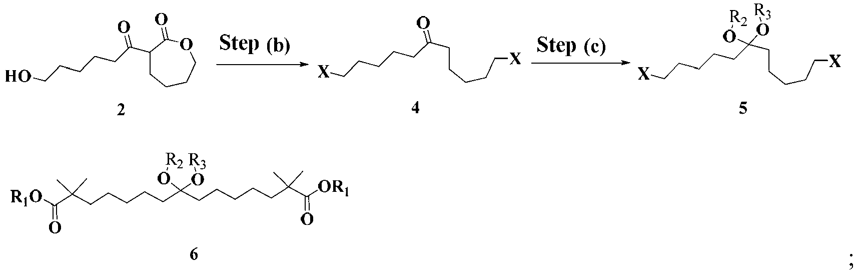
or comprising the following steps:
(b') hydrolyzing Compound 2 to form Compound 3,
(b") reacting Compound 3 with a halogenating agent to obtain Compound 4,
(c) reacting Compound 4 with an alcohol to produce Compound 5, and
(d) reacting Compound 5 with an iso-butyrate to obtain Compound 6,
wherein the reaction scheme is as follows:

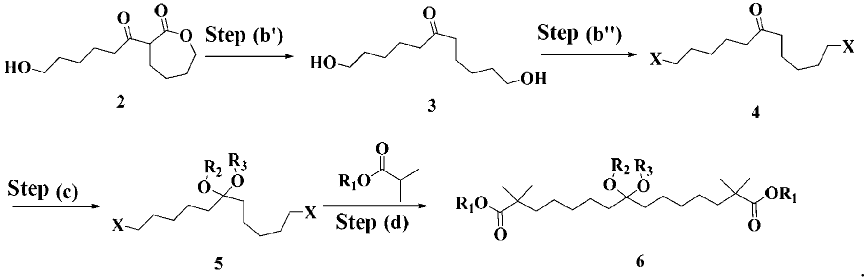

21. A method for preparing Compound 4, comprising the following steps:
(a) subjecting Compound 1 to self-condensation to obtain Compound 2, and
(b) reacting Compound 2 with a halogenating agent to obtain Compound 4,
wherein the reaction scheme is as follows: f>o 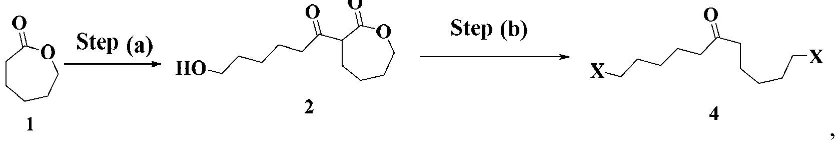
in which X is selected from Cl, Br or I, and more preferably Br;
or comprising the following steps:
(a) subjecting Compound 1 to self-condensation to obtain Compound 2,
(b') hydrolyzing Compound 2 to form Compound 3, and
(b") reacting Compound 3 with a halogenating agent to obtain Compound 4, wherein the reaction scheme is as follows: 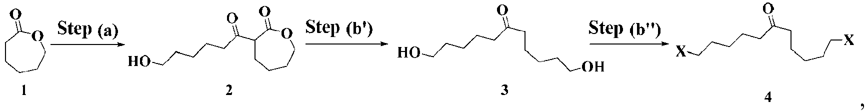
in which X is selected from Cl, Br or I, and more preferably Br.
